(19) Europäisches Patentamt
European Patent Office
Office européen des brevets

(11) Publication number: 0 125 698 B1

# (12) EUROPEAN PATENT SPECIFICATION

(45) Date of publication of patent specification: 13.12.89

(51) Int. Cl.[4]: C 12 Q 1/04, C 12 Q 1/26, C 12 Q 1/54

(21) Application number: 84105568.4

(22) Date of filing: 16.05.84

## (54) A process for screening microorganisms producing glucose-2-oxidase.

(30) Priority: 16.05.83 US 495193
16.05.83 US 495194

(43) Date of publication of application:
21.11.84 Bulletin 84/47

(45) Publication of the grant of the patent:
13.12.89 Bulletin 89/50

(84) Designated Contracting States:
AT BE CH DE FR GB IT LI LU NL SE

(56) References cited:
EP-A-0 091 837
EP-A-0 098 533
FR-A-2 504 679
US-A-4 017 420
US-A-4 246 347

(73) Proprietor: NABISCO BRANDS, Inc.
Nabisco Brands Plaza
Parsippany New Jersey 07054 (US)

(72) Inventor: Horwath, Robert O.
223 Bayberry Lane
Westport, Conn. (US)

(74) Representative: Brauns, Hans-Adolf, Dr. rer. nat. et al
Hoffmann, Eitle & Partner, Patentanwälte
Arabellastrasse 4
D-8000 Munich 81 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 125 698 B1

## Description

This invention relates to the field of microbiology and more particularly to the selection and screening of microorganisms.

A variety of approaches has been used to improve the economy of biologically-based industrial processes by "improving" the organism involved. These techniques constitute what may be categorized as strain improvement programs. The efficacy of improving said processes is dependent on the type of organism and the nature of the end-product.

The success of any strain improvement program will be directly affected by the facility with which genetic diversity can be generated in the subject organism, or alternatively the ease with which the genetic diversity already present in nature can be evaluated.

A colony that appears on agar medium following plating out of spores, cells, or small hyphal fragments consists of a population of cells most of which are genetically identical, although some cells may differ due to spontaneous mutation during the growth of the colony or to nuclear heterogeneity in the original propagule.

It was the rare occurrence of spontaneous mutations within existing cultures that provided the major source of strain improvement germplasm in the early years of the fermentation industry. A secondary source of improved strains was nature itself, that is, the isolation from nature of previously unknown strains with improved characteristics.

As a fuller understanding of the biological and chemical basis of genetic change developed, strain improvement programs incorporated this new knowledge into their rationale. For example, induced mutagenesis to generate genetic diversity followed by the subsequent screening, selection and purification of superior strains represents one of the most effective means of improving the yield of a fermentation product. Mutation programs are vital to the fermentation industry in that higher productivities exhibited by the new strains are essential in reducing costs.

It is now appreciated that the choice of a particular mutagen as well as the actual conditions of mutagenesis can play a major role in determining the types and numbers of mutants recovered during a strain improvement program. In general, two experimental approaches have been used to recover new strains resulting from induced mutagenesis experiments; these are: screening and selection.

In a screening system all strains grow with the exception of those killed outright as a result of the mutagenesis treatment; thus each isolate must be examined to identify the desired characteristic. Since tens of millions of isolates must be examined, this approach can be highly labor intensive.

In a selection system, the experimental conditions are chosen so as to establish a growth differential between the rare strains possessing the desired characteristic and all other strains which do not possess said trait. In certain instances the selected strain will not grow under the conditions of the experiment while the non-selected strains will grow. Thus, by removing the growing strains by filtration or other means, the size of the population of cells remaining to be examined is dramatically reduced. Alternatively, conditions may be established such that the selected strain will grow while the non-selected strains are inhibited, here again effectively reducing the population to be examined.

Although induced mutagenesis has been an extremely powerful force in the area of strain improvement, there are some limitations. For example, as more and more mutations are accumulated in a strain as a result of the continuing improvement program, a saturation level is reached. Subjecting such a strain to further selection often results in a loss of productivity due to reversion of existing mutations.

A more fundamental limitation exists in induced-mutation based improvement programs, namely, such programs are based on the assumption that the strains possess the activity to be improved. In other words, the organism must possess, in its genetic repertoire, the information to direct the synthesis of a gene product before any genetically-based improvement program relating to the function of the product may be considered.

A variety of genetic approaches has been developed to reduce these limitations. For example, hybridization techniques allow for genetic recombination to occur among a number of different strains. Hybridization can be achieved by means of sexual reproduction or asexual processes such as somatic cell fusion or heterokaryon formation. The advent of recombinant DNA technology has reduced the limitations on improvement programs even further. The ability to transfer genes between organisms of widely divergent genetic backgrounds has provided the experimenter with a virtually limitless supply of genetic information upon which to improve. This advent of genetic engineering technology has prompted a renewed interest in natural sources of genetic variability, not with a view toward isolating and developing new strains, *per se,* but rather as a source of as little as a single gene which may be transferred to already established strains.

Regardless of the source of the variant strain, be it either nature, a spontaneous mutation, an induced mutation, or a recombinant resulting from sexual, asexual or genetic engineering processes, methods of screening and selection remain of critical importance, allowing the experimenter to recover the variant strain from among the population of existing strains from which it arose.

In light of the subject invention, one group of organisms of particular interest with regard to strain improvement programs are those useful for the isomerization of glucose to fructose.

Most food grade glucose is provided as an enzymatic hydrolysate of corn starch, i.e., the corn syrup of

commerce. Glucose is generally rated at being 60 to 80% as sweet as sucrose and therefore sells at a correspondingly lower price. It has long been known to isomerize glucose to fructose, which is even sweeter than sucrose, by employing an enzyme having glucose isomerase activity. Preferably, such an enzyme is one which has been immobilized onto insoluble supports, such as by crosslinking the enzyme with the support matrix or entrapment in a polymer matrix support such as diethylaminoethyl cellulose or porous glass. The isomerization of glucose provides an equilibrium mixture typically containing 42—50% fructose and is referred to as high fructose corn syrup (HFCS).

Recently, it has been proposed to achieve substantially complete conversion of glucose to fructose by first enzymatically converting glucose to glucosone and thereafter chemically reducing the glucosone to fructose. Thus, in accordance with U.S. Patent No. 4,246,347, at least about ninety-five percent of D-glucose in aqueous solution is enzymatically oxidized to D-glucosone employing an enzyme having glucose-2-oxidase activity, preferably one obtained from *Polyporus obtusus* or *Aspergillus oryzae,* while removing or utilizing co-produced hydrogen peroxide, the D-glucosone being thereafter hydrogenated to D-fructose. As is known in the art, the glucose-2-oxidase obtained from *Polyporus obtusus,* the preferred organism up to the present, is employed in the form of a cell-free extract, primarily because only low enzyme activity is obtained when mycelia of this organism are used as the source of the enzyme.

These conversions, D-glucose to D-glucosone and D-glucosone to D-fructose, can be regarded as preceeding in accordance with the following equations:

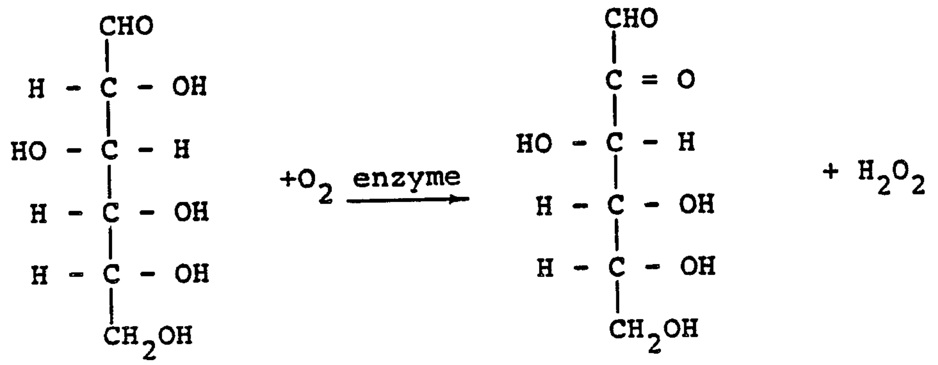


D-glucose

D-glucosone
(D-arabino-2-hexosulose)

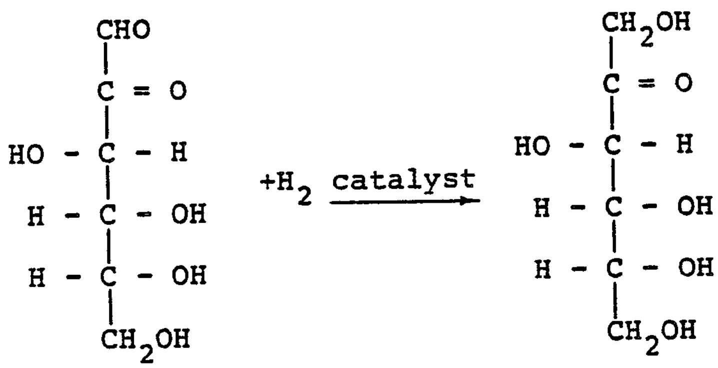


D-glucosone
(D-arabino-2-hexosulose)

D-fructose

Recently, it has been disclosed by Horwath in U.S. Patent No. 4,442,207 that various species of Basidiomycetes produce significant quantities of glucose isomerase and glucose-2-oxidase. These findings, particularly when taken in light of the methods of fructose production as described above, warranted the development of a large scale, efficient screening system for the recovery of glucose-2-oxidase producing strains. It is the principle object of the instant invention to provide such a screening system.

This invention relates to a rapid screening method for the detection of increased or decreased production of glucose-2-oxidase by any microorganism.

The invention comprises a process for screening micro-organisms for the production of the enzyme glucose-2-oxidase which comprises the steps of:

(a) forming a screening plate comprising a suspension of said microorganisms on a solid or semi-solid medium which promotes the growth of said microorganisms and provides a suitable substrate for the catalytic activity of the enzyme;

(b) incubating said inoculated medium under condittions that promote the synthesis of glucose-2-oxidase; and

(c) identifying *in situ* those colonies expressing glucose-2-oxidase activity by detecting the presence of a product of glucose-2-oxidase catalysis surrounding each colony by reaction with an analytically indicatable reagent.

The microorganism is plated onto the surface of a solid or semi-solid medium. The medium contains all of the basic nutritional requirements of the particular strain as well as any specific factors which may be required to promote the synthesis of or aid in the detection of the desired enzyme. The medium surrounding each colony is assayed for the presence of an enzyme product. Assays particularly useful with regard to the subject invention are those which are non-destructive to the microbial colony. However, toxic reagents may be employed for periods of short exposure in the evaluation of the enzyme activity, when the analytical reaction of said reagent is rapid thus ensuring that a few viable cells are likely to remain in the colony even after treatment with the toxic reagent. Thus, the colony producing the enzyme can be used directly as a source of cells for isolation and further evaluation, eliminating the necessity of "replica plating" each screening plate for the purpose of strain maintenance.

The subject invention is particularly useful in that it provides for the early detection of glucose-2-oxidase-producing microrganisms.

According to one embodiment of the invention a soil sample containing microorganisms to be screened is plated onto a culture medium which promotes the growth of said microorganisms and the synthesis of glucose-2-oxidase by said microorganisms. After a period of time sufficient to allow development of the organisms and their attendant enzyme activity, an overlay is applied to the screening plates. The overlay, in addition to containing additional substrate for glucose-2-oxidase, may also provide for the stabilization of one of the products of glucose-2-oxidase activity, namely glucosone. After a sufficient period of time has elapsed for the accumulation of said enzymatic products of glucose-2-oxidase activity, an analytically indicatable reagent is added to the overlayed screening plate and those miroorganisms capable of glucose-2-oxidase synthesis are identified by a positive reaction of the indicator reagent. The microorganisms may then be recovered from those areas of the screening plates displaying a positive reaction of the indicator reagent, sub-cultured and the presence of glucose-2-oxidase reconfirmed.

In a second embodiment of the present invention, the process for screening microorganisms for the production of glucose-2-oxidase comprises the steps of forming a screening plate comprising a suspension of said microorganisms on a solid or semi-solid medium which promotes the growth of said microorganisms and provides a substrate for the production of hydrogen peroxide by said enzyme; incubating said inoculated medium under conditions that promote the synthesis of glucose-2-oxidase; and identifying *in situ* those colonies expressing glucose-2-oxidase activity by detecting the presence of hydrogen peroxide by reaction with analytically indicatable reagent.

In this second embodiment a soil sample containing microorganisms to be screened is plated onto a culture medium containing sorbose. The sorbose, in addition to serving as a carbon source for the growth of the desired organism, also serves as a substrate for glucose-2-oxidase. As the organism metabolizes the sorbose by means of glucose-2-oxidase, one of the products of said metabolism, hydrogen peroxide is liberated into the medium. Hydrogen peroxide-producing colonies can then be identified by reaction with appropriate analytically indicatable reagents and $H_2O_2$ producing colonies isolated from the culture plates. This process is particularly useful in that it reduces the number of false positive colonies by eliminating a source of competing enzyme activity.

One criterion of strain improvement is a change in the activity and/or amount of a particular enzyme produced by an organism. The subject invention exploits this feature to establish a rapid, inexpensive and non-labor intensive method for the *in situ* screening of glucose-2-oxidase-producing microorganisms. In a preferred embodiment of the invention, a population of microorganisms to be tested is plated onto a solid growth medium. Microorganisms suitable for screening according to the subject invention include: bacteria, actinomycetes, fungi and unicellular algae, although the invention is particularly useful for screening fungi of the Basidiomycete class.

Basidiomycete fungi are relatively slow-growing micro-organisms; a period of time from 3—7 days from the initiation of germination to visible colony formation is not uncommon. This protracted developmental period makes the screening and selection of Basidiomycetes from nature particularly difficult. If, for example, soil sample containing Basidiomycetes and other soil microbes is plated onto a screening medium and incubated for 3—7 days to allow for the development of the Basidiomycete colonies, the plates would be completely overgrown with contaminating, faster growing microbes present in the soil sample.

In light of the fact that Basidiomycete fungi have been shown to produce glucose-2-oxidase and said enzyme is useful in the conversion of glucose to fructose (Horwath U.S. Patent No. 4,442,207) a method for the large scale, efficient screening of Basidiomycete fungi for the production of glucose-2-oxidase would be

clearly useful. However, because of the slow growth displayed by Basidiomycetes as discussed above, the early detection of enzyme activity in said fungi during the screening procedure is a necessity. The ability to detect enzyme activity in the subject organism early in its developmental history is not only useful in the screening of soil samples (e.g., preventing overgrowth of contaminants), but even when screening pure cultures of Basidiomycetes for strain improvement purposes since the reduction of the 3—7 day development period certainly would be an advantage.

Facilitating the early detection of Basidiomycetes expressing glucose-2-oxidase, it has been discovered that the germinating fungi produce measurable amounts of the desired enzyme. Thus, according to a preferred embodiment of the invention, at least one product of the glucose-2-oxidase reaction is detected in the area surrounding the developing organism by reaction with a suitable analytically indicatable reagent. The detected reaction product acts as a "chemical microscope" permitting the detection of glucose-2-oxidase producing cells long before such cells multiply to form discrete visible colonies.

In the case of spore-producing Basidiomycetes, by virtue of liberating a reaction product of glucose-2-oxidase activity in the area surrounding said spores, a "metabolic shadow" is cast which may be detected by the appropriate reagents. Thus, by recovering germinating spores from the area indicated by a positive indicator reaction, said germinating spores may be isolated quite early in their developmental history.

In light of the subject invention, the enzyme reaction of interest may be represented as follows:

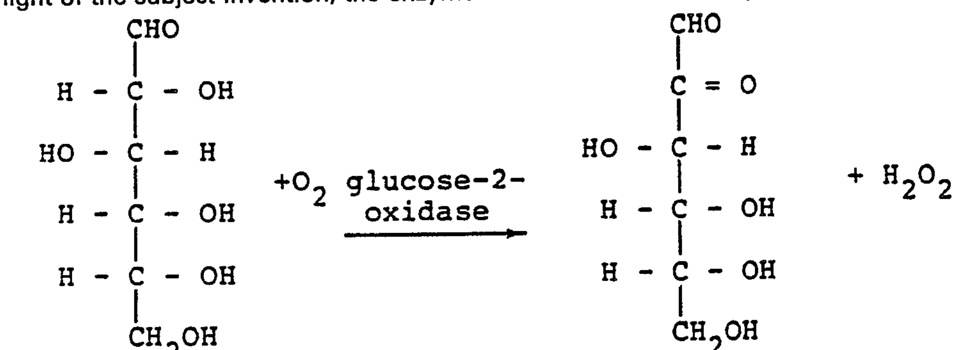


D-glucose D-glucosone (D-arabino-2-hexosulose)

Due to the fact that glucosone is a unique product of glucose-2-oxidase activity as contrasted with hydrogen peroxide which may result from unrelated enzyme activity, the measurement of glucosone is a direct correlate of glucose-2-oxidase activity.

According to one embodiment of the invention a said sample is plated onto a solid screening medium, incubated for a sufficient period of time to allow for the development of glucose-2-oxidase containing microorganisms and the expression of said enzyme by said organism. This time period may range from about 24 to about 96 hours depending on a number of parameters such as the concentration and composition of the microorganisms within the soil sample. In general, an incubation period of about 72 hours is preferred.

After the initial incubation period has elapsed, if glucosone is the product to be detected, the plates are overlayed with a second medium comprising additional substrate (e.g. glucose) for glucose-2-oxidase and preferably containing a glucosone-stabilizing substance such as sodium fluoride. The presence of sodium fluoride inhibits the metabolism of glucosone and permits the product to accumulate within the microorganism and in the surrounding medium. The labile product is thus metabolically sequestered and may be identified more readily by reaction with a suitable analytically indicatable reagent.

After a sufficient period of time to allow for the accumulation of glucosone, said period of time being from about 12 to about 24 hours, preferably about 16 hours; the overlaid screening plates are assayed for the presence of glucosone by the addition of a suitable analytically indicatable reagent.

A useful reagent in this regard has been found to be 2,4-dinitrophenylhydrazine. It forms a hydrazone with glucosone resulting in a red-orange precipitate surrounding or developing on the glucose-2-oxidase producing organisms. Although it has been found that approximately 20% of the positive reactions are due to hydrazone derivatives other than from glucosone, the presence of the glucosone derivative is easily confirmed by extraction into ethyl acetate and analysis by high pressure liquid chromatography (HPLC).

According to one embodiment of the invention, each hydrazone-positive area of the screening plate is "double-plugged", one sample being subjected to HPLC analysis to confirm the presence of the glucosone derivative while the remaining sample is retained for purpose of isolating and sub-culturing the microorganism.

In order to reduce the number of false positive reactions of the indicator reaction, the subject invention also provides for the use of specially selected carbon sources. For example, if glucose, the natural substrate of glucose-2-oxidase, is employed, a variety of false positive reactions will occur due to the metabolism of glucose by other hydrogen peroxide liberating enzymes, (e.g., glucose-1-oxidase, which catalyzes the oxidation of glucose to glucuronic acid and $H_2O_2$). Because the glucose-2-oxidase will accept other carbohydrate substrates not utilized by the competing glucose-1-oxidase, such substrates are employed to

reduce false positive reactions. Suitable sole-carbon sources, which reduce false positive reactions; include substrates such as sorbose or xylose. Sorbose or xylose may be employed alone or in combination, although due to a lower cost and a more favourable rate of reaction, sorbose is preferred.

Sorbose is converted into 5'-keto-fructose liberating $H_2O_2$ in the presence of glucose-2-oxidase whereas sorbose is not an acceptable substrate for glucose-1-oxidase. Furthermore, the presence of sorbose in the screening plates promotes the formation of, but restricts the size of colonial growth of certain microorganisms, thus restricting the spread of said microorganisms. Colony-size limitation is of additional advantage in that a larger number of colonies may be evaluated per plate.

The quantity of sorbose, or xylitol employed usually ranges from about 0.1 to about 5%, preferably from 1% to 3%.

To further illustrate the present invention, the following exemplification is provided.

Example 1

This example illustrates the screening of soil samples for glucose-2-oxidase producing microorganisms by employing an overlay method to detect the presence of glucosone.

A soil sample is inoculated onto a large screening plate (1.5 liter of medium/plate) and incubated for 72 hours at 25°C.

The base layer medium is prepared as follows: All percentages (w/v).

| | |
|---|---|
| glucose | 1% |
| peptone | 1% |
| agar | 1.5% |
| pH | 5.8 |

After the incubation period, the plates are overlayed with 0.5 liters of a medium comprising:

| | |
|---|---|
| glucose | 4% |
| NaF | 0.02M |
| agar | 1.5% |
| pH | 7.0 |

The overlayed plates are incubated for 16 hours at 25°C after which time the plates are analyzed for the presence of glucosone by reaction with 2, 4-dinitrophenylhydrazine.

The 2,4-dinitrophenylhydrazine (2,4-DNPH) is prepared by dissolving 80 gms of 2,4-DNPH in 400 ml of concentrated $H_2SO_4$; · 600 ml of $H_2O$ is added slowly and carefully with stirring followed by the addition of 2000 ml of 95% ethanol. The solution is filtered using a Buchner funnel and diluted 1:3 for spraying onto agar plates.

The plates are sprayed with 2,4-DNPH reagent and after one hour, an orange-red zone will develop surrounding or on glucose-2-oxidase-producing organisms as a result of the glucosone reaction with the reagent.

Samples are then removed from the areas of positive reaction and the presence of glucosone-DNPH derivative is confirmed by high pressure liquid chromatography and the desired organism isolated and sub-cultured.

Example 2

This example illustrates the screening of soil samples for glucose-2-oxidase-producing microorganism by a spraying method to detect the presence of glucosone.

The base layer medium is prepared, inoculated and incubated as described in Example 1.

After 72 hours, the base layer is sprayed with a medium comprising:

| | |
|---|---|
| glucose | 6% |
| NaF | 0.02M |
| agar | 0.5% |
| pH | 7.0% |

After incubation for 16 hours at 25°C the sprayed plates are analyzed for the presence of glucosone as described in Example 1.

Example 3

This example illustrates the screening of soil samples for glucose-2-oxidase-producing microorganisms by detection of hydrogen peroxide.

A sample is inoculated onto a large screening plate (1.5 liter of medium/plate) and incubated for 48—72 hours at 25°C.

The medium is prepared as follows: All percentages are (w/v).

| | |
|---|---|
| sorbose | 2.0% |
| peptone | 1.0% |
| NaF | 1mM |
| agar | 2.0% |
| pH | 5.8 |

After the incubation period, the plates are sprayed with the hydrogen peroxide indicator reagent.

The indicator reagent is prepared by mixing 2 parts of ABTS stock solution* with 3 parts peroxidase stock solution.**

* ABTS STOCK SOLUTION
ABTS — 2.65 (w.v) in water
ABTS is the leuco dye
2,2-azino-di-(3-ethyl-benzthiazoline sulfonate).

** PEROXIDASE STOCK SOLUTION
100 units of horseradish peroxidase/ml
water (Millipore filtered).

The plates are incubated at 30°C for up to an additional 24 hours. The formulation of a purple zone indicates the presence of a glucose-2-oxidase producing microorganism.

Alternatively, the ABTS and peroxidase may be combined with agar and applied as an overlay.

**Claims**

1. A process for screening microorganisms for the production of the enzyme glucose-2-oxidase which comprises the steps of:

(a) forming a screening plate comprising a suspension of said microorganisms on a solid or semi-solid medium which promotes the growth of said microorganisms and provides a suitable substrate for the catalytic activity of the enzyme;

(b) incubating said inoculated medium under conditions that promote the synthesis of glucose-2-oxidase; and

(c) identifying *in situ* those colonies expressing glucose-2-oxidase activity by detecting the presence of a product of glucose-2-oxidase catalysis surrounding each colony by reaction with an analytically indicatable reagent.

2. The process according to Claim 1 including the further step of recovering the so-identified microorganism from said screening plate.

3. The process according to Claim 1 wherein said microorganism is a bacterium, an actinomycete, a fungus or a unicellular alga.

4. The process according to Claim 3 wherein said microorganism is a Basidiomycete.

5. The process according to any of Claims 1—4 wherein the product is glucosone.

6. The process according to Claim 5 wherein said growth promoting enzyme substrate is glucose in a concentration of from about 1 to about 6%.

7. The process according to Claim 5 or 6 wherein said glucose is present in a concentration of about 2%.

8. The process according to any of Claims 5—7 wherein the reagent comprises 2,4-dinitrophenylhydrazine.

9. The process according to any of Claims 1—4 wherein the product is hydrogen peroxide.

10. The process according to Claim 9 wherein said medium is formed with growth promoting, suitable substrates selected from the group comprising xylose or sorbose.

11. The process according to Claim 10 wherein said medium comprises from about 0.5% to about 5% w/v sorbose.

12. The process according to Claim 11 wherein said medium comprises about 2% w/v sorbose.

13. The process according to any of Claims 9—12 wherein the reagent comprises horseradish peroxidase.

**Patentansprüche**

1. Verfahren zum Screenen von Mikroorganismen für die Herstellung des Enzyms Glucose-2-oxidase, umfassend folgende Stufen:

(a) Bildung einer "Screening"-Platte aus einer Suspension der Mikroorganismen auf einem festen oder halbfesten Medium, welches das Wachstum der Mikroorganismen unterstützt, und ein geeignetes Substrat für die katalytische Aktivität des Enzyms darstellt,

(b) Inkubieren des inokulierten Mediums unter Bedingungen, bei welchen die Synthese von Glucose-2-oxidase eintritt, und

(c) in situ-Identifizieren von solchen Kolonien, die eine Glucose-2-oxidase-Aktivität ausdrücken, indem man die Gegenwart eines Produktes der Glucose-2-oxidase-Katalyse, welches jede der Kolonien umgibt, durch Umsetzen mit einem analytisch anzeigenden Reagens nachweist.

2. Verfahren gemäss Anspruch 1, welches die weitere Stufe einschliesst, dass man die so identifizierten Mikroorganismen von der Screening-Platte gewinnt.

3. Verfahren gemäss Anspruch 1, bei dem der Mikroorganismus ein Bakterium, ein Actinomycet, ein Fungus oder eine einzellige Alge ist.

4. Verfahren gemäss Anspruch 3, bei dem der Mikroorganismus ein Basidiomycet ist.

5. Verfahren gemäss einem der Ansprüche 1 bis 4, bei dem das Produkt Glukoson ist.

6. Verfahren gemäss Anspruch 5, bei dem das wachstumsbeschleunigende Enzymsubstrat Glucose in einer Konzentration von etwa 1 bis etwa 6% ist.

7. Verfahren gemäss Anspruch 5 oder 6, bei dem die Glucose in einer Konzentration von etwa 2% vorliegt.

8. Verfahren gemäss einem der Ansprüche 5 bis 7, bei dem das Reagens 2,4-Dinitrophenylhydrazin umfasst.

9. Verfahren gemäss einem der Ansprüche 1 bis 4, bei dem das Produkt Wasserstoffperoxid ist.

10. Verfahren gemäss Anspruch 9, bei dem das Medium mit wachstumsbeschleunigenden geeigneten Substraten, ausgewählt aus der Gruppe, bestehend aus Xylose oder Sorbose, ausgebildet ist.

11. Verfahren gemäss Anspruch 10, bei dem das Medium etwa 0,5 bis etwa 5% G/V Sorbose umfasst.

12. Verfahren gemäss Anspruch 11, bei dem das Medium etwa 2% G/V Sorbose umfasst.

13. Verfahren gemäss einem der Ansprüche 9 bis 12, bei dem das Reagens Meerrettichperoxidase.

**Revendications**

1. Procédé pour le criblage de microorganismes pour la production de l'enzyme glucose-2-oxydase, qui comprend les étapes consistant à:

(a) former une plaque de criblage comprenant une suspension desdits microorganismes sur un milieu solide ou semi-solide qui favorise la croissance desdits microorganismes et fournit un substrat approprié pour l'activité catalytique de l'enzyme;

(b) incuber ledit milieu inoculé dans des conditions qui favorisent la synthèse de la glucose-2-oxydase; et

(c) identifier *in situ* les colonies exprimant l'activité glucose-2-oxydase, par détection de la présence d'un produit de catalyse par la glucose-2-oxydase entourant chaque colonie par réaction avec un réactif d'indication analytique.

2. Procédé selon la revendication 1, comprenant l'étape supplèmentaire consistant à récupérer le microorganisme ainsi identifié à partir de ladite plaque de criblage.

3. Procédé selon la revendication 1, dans lequel ledit microorganisme est une bactérie, un actinomycète, un champignon ou une algue unicellulaire.

4. Procédé selon la revendication 3, dans lequel ledit microorganisme est un Basidiomycète.

5. Procédé selon l'une des revendications 1—4, dans lequel le produit est la glucosone.

6. Procédé selon la revendication 5, dans lequel ledit substrat d'enzyme favorisant la croissance est le glucose à une concentration allant d'environ 1 à environ 6%.

7. Procédé selon l'une des revendications 5 ou 6, dans lequel ledit glucose est présent à une concentration d'environ 2%.

8. Procédé selon l'une des revendications 5—7, dans lequel le réactif comprend la dinitro-2,4 phénylhydrazine.

9. Procédé selon l'une des revendications 1—4, dans lequel le produit est le peroxyde d'hydrogène.

10. Procédé selon la revendication 9, dans lequel ledit milieu est formé avec des substrats appropriés favorisant la croissance, choisis dans le groupe comprenant le xylose ou le sorbose.

11. Procédé selon la revendication 10, dans lequel ledit milieu comprend d'environ 0,5% à environ 5% p/v de sorbose.

12. Procédé selon la revendication 11, dans lequel ledit milieu comprend environ 2% p/v de sorbose.

13. Procédé selon l'une des revendications 9—12, dans lequel le réactif comprend la peroxydase de raifort.